# FASCICULE DE BREVET EUROPEEN

(11) **EP 4 475 918 B1**
(45) Date de publication et mention de la délivrance du brevet: **11.03.2026**
(21) Numéro de dépôt: 23703268.5
(22) Date de dépôt: 09.01.2023
(51) Int. Cl.: A61M 11/00, A61M 15/00, A61M 15/08, B05B 11/00, A61J 1/14, B65D 50/00, A61J 1/06, A61J 1/16, A61J 7/00, B05B 11/10

(54) **ENSEMBLE COMPRENANT UN DISTRIBUTEUR DE PRODUIT FLUIDE ET UN DISPOSITIF DE DEVERROUILLAGE**
ANORDNUNG MIT EINEM SPENDER FÜR EIN FLÜSSIGES PRODUKT UND EINER ENTRIEGELUNGSVORRICHTUNG
ASSEMBLY COMPRISING A FLUID PRODUCT DISPENSER AND A UNLOCKING DEVICE

(30) Priorité: 11.01.2022 FR 2200188
(43) Date de publication de la demande: 18.12.2024
(73) Titulaire: APTAR France SAS, 27110 Le Neubourg (FR)
(72) Inventeur: FABIEN, David, 29290 Saint Renan (FR)
(74) Mandataire: CAPRI
(86) Numéro de dépôt international: PCT/FR2023/050020
(87) Numéro de publication internationale: WO 2023/135383

(56) Documents cités:
- EP-A1- 2 436 415
- EP-A2- 0 071 931
- WO-A1-01/93932
- WO-A1-2018/058287
- WO-A1-2021/084092
- DE-U1- 202010 005 002
- US-A- 5 692 492

## Description

La présente invention concerne un ensemble comprenant deux entités distinctes adaptées à être rapportées l'un sur ou en contact de l'autre, afin de coopérer pour générer une opération de déverrouillage, ces deux entités incluant d'une part un distributeur de produit fluide comprenant un organe mobile et un système de blocage commutable entre une position de blocage dans laquelle l'organe mobile est bloqué et une position de libération dans laquelle l'organe mobile est libre, et d'autre part, un dispositif de déverrouillage, distinct du distributeur, apte à commuter le système de blocage entre la position de blocage et la position de libération. Le domaine d'application privilégié de cette invention est celui de la pharmacie, sans pour autant exclure d'autres domaines, comme ceux de la parfumerie, des cosmétiques ou même de la droguerie.

De manière imagée, on peut dire que le distributeur intègre une serrure bloquant une de ses fonctions et que le dispositif de déverrouillage est une sorte de clé qui permet d'actionner la serrure, afin de débloquer la fonction du distributeur. Cette fonction peut être de toute nature, mais toujours commandée par un organe mobile, qui permet par exemple d'accéder à un composant du distributeur ou d'autoriser le fonctionnement du distributeur. L'organe mobile peut être un capot, un couvercle, une trappe, un fond ou encore une partie d'un mécanisme intervenant dans la distribution du produit fluide. L'organe mobile peut permettre l'accès à un réservoir, par exemple équipé d'un organe de distribution, comme une pompe, une valve ou un simple bouchon. L'organe mobile peut permettre de contrôler la posologie (temps entre deux prises de médicament). La nature et la fonction de l'organe mobile n'est pas critique pour la présente invention, dans la mesure où son blocage empêche une utilisation non indiquée du distributeur.

Dans le domaine de la pharmacie par exemple, l'administration de substances puissantes, potentiellement léthales, chez l'homme peut être une nécessité dans certaines situations. C'est notamment le cas pour le traitement de pathologies particulières ou encore pour les personnes ayant besoin de traitements palliatifs dans des contextes de fin de vie.

La manipulation de telles substances requiert une grande prudence et des dispositifs d'administrations extrêmement sûrs, pour éviter les risques d'overdose, qui peuvent survenir en cas d'administration d'un nombre trop important de doses consécutives.

Un risque est également présent, qui concerne l'utilisation des dispositifs par une personne autre que la personne à qui le traitement est destiné (enfants par exemple).

Dans ce contexte, l'objectif de l'invention est de développer un ensemble distributeur/dispositif de déverrouillage sécurisant pour éviter les risques d'overdose, et utilisable uniquement par la personne en ayant le besoin. Ainsi, seule la personne possédant ce dispositif de déverrouillage dédié peut utiliser le distributeur. Le dispositif de déverrouillage peut être utilisé en usine ou par une entité habilitée, comme un pharmacien ou un docteur.

Dans l'art antérieur, on connait déjà des dispositifs de déverrouillage permettant de désactiver ou neutraliser des antivols magnétiques, comme ceux que l'on utilise dans les magasins, par exemple pour les vêtements. Ce type d'antivol magnétique est facilement désactivable au moyen d'un simple aimant permanent qui l'on rapporte sur l'antivol, de sorte qu'il n'est pas sûr.

On connait aussi de nombreux systèmes de verrouillage qui sont désactivables au moyen d'une clé spécifique, qui agit mécaniquement sur le système de verrouillage. L'inconvénient de ces systèmes mécaniques est qu'ils sont visibles et peuvent donc faire l'objet de tentative d'effraction, qui peuvent conduire à l'ouverture du système de verrouillage ou à sa détérioration.

Ainsi, les antivols magnétiques sont inaccessibles, mais trop simples à désactiver et les antivols mécaniques sont plus difficiles à désactiver, mais trop accessibles.

Les documents DE 20 2010 005 002 U1, US 5 695 492 A et EP 0 071931 A2 décrivent des exemples de dispositifs de verrouillage mécaniques impliquant, en outre, des forces magnétiques.

La présente invention a pour but de remédier aux inconvénients des systèmes de verrouillage de l'art antérieur, en cumulant leurs avantages et en éliminant leurs inconvénients. Le système de blocage de l'invention doit être à la fois inaccessible et difficile à désactiver.

Pour ce faire, la présente invention, telle que définie dans les revendications énoncées, propose un ensemble comprenant :
- un distributeur de produit fluide comprenant un organe mobile et un système de blocage commutable entre une position de blocage dans laquelle l'organe mobile est bloqué et une position de libération dans laquelle l'organe mobile est libre,
- un dispositif de déverrouillage, distinct du distributeur, apte à commuter le système de blocage entre la position de blocage et la position de libération, ce dispositif de déverrouillage agissant sans contact, à distance, par génération de courant de Foucault. Au lieu d'utiliser la simple attraction magnétique, comme dans un antivol magnétique classique, la présente invention utilise un principe électromagnétique basé sur les courants de Foucault et la loi de Lenz-Faraday.

On appelle « courants de Foucault » les courants électriques créés dans une masse conductrice, soit par la variation au cours du temps d'un champ magnétique extérieur traversant ce milieu (le flux du champ à travers le milieu), soit par un déplacement de cette masse dans un champ magnétique. Ils sont une conséquence de l'induction électromagnétique.

Lorsque la variation de flux est due à un déplacement du milieu devant un champ magnétique constant, les courants de Foucault sont responsables de l'apparition de forces de Laplace qui s'opposent au déplacement, d'où l'effet de freinage observé sur les systèmes utilisant ce genre de dispositif.

Les forces de Laplace, créées par ce phénomène de courant induits, s'opposent à la cause qui leur a donnée effet c'est-à-dire la rotation de(s) aimant(s) autour de l'axe.

Pour s'opposer à ce mouvement relatif de rotation, les forces de Laplace prennent donc la forme d'un couple sur le disque d'induction visant à faire tourner le disque dans le même sens de rotation que l'axe des aimants afin de réduire ces courants.

En physique, la loi de Lenz-Faraday, ou loi de Faraday, permet de rendre compte des phénomènes macroscopiques d'induction électromagnétique. Le sens du courant induit (orienté dans le même sens que le champ électrique induit) est tel que celui-ci tend toujours à s'opposer, par ses effets, à la cause qui l'a produit :
- dans le cas d'un champ magnétique variable, le champ créé par le courant induit lui-même s'oppose à la variation du champ initial,
- dans le cas d'un circuit mobile, les forces de Laplace dues au courant induit s'opposent au mouvement initial du circuit.

Cette interprétation est connue sous le nom de loi de modération de Lenz.

Le système de blocage comprend un élément d'induction mobile et le dispositif de déverrouillage comprend des moyens de génération de champ magnétique variable induisant une force électromotrice sur l'élément d'induction mobile qui permet de commuter le système de blocage de sa position de blocage vers sa position de libération. L'élément d'induction peut être déplacé en rotation, en translation ou en pivotement, selon la nature du champ magnétique variable. Dans l'invention revendiquée, l'élément d'induction est déplacé en rotation. Dans une forme non revendiquée, un coulisseau conducteur amagnétique est déplacé en translation de manière à libérer l'organe mobile.

L'élément d'induction mobile, selon l'invention, est un disque d'induction rotatif autour d'un axe X et les moyens de génération de champ magnétique variable comprennent au moins un aimant permanent entrainé en rotation autour d'un axe Y ou un jeu de solénoïdes disposés autour d'un axe Y et alimentés de manière alternée de façon à réaliser un champ magnétique tournant, les axes X et Y étant alignés lorsque le dispositif de déverrouillage est rapporté sur le distributeur ou vice versa, de sorte les moyens de génération de champ magnétique variable induisent ainsi un mouvement de rotation au disque d'induction à partir d'une position de repos vers une position active.

Comparé au système de freinage des camions, dans lequel le disque d'induction tourne et des aimants statiques ralentissent la rotation du disque d'induction, dans la présente invention, les aimants tournent et font tourner le disque d'induction qui est statique au repos.

Avantageusement, le disque d'induction est sollicité en position de repos par des moyens élastiques. Ainsi, il n'est pas nécessaire d'utiliser le dispositif de déverrouillage pour ramener le disque d'induction en position initiale de repos. De préférence, le disque d'induction peut comprendre au moins un profil de butée pour limiter sa rotation entre la position de repos et la position active.

Le disque d'induction peut constituer à lui seul ou presque le système de blocage et agir directement sur l'organe mobile, mais de préférence, le système de blocage comprend en outre un loquet déplaçable entre une position d'interposition dans laquelle le loquet bloque l'organe mobile et une position d'effacement dans laquelle l'organe mobile est libre, le loquet étant bloqué dans la position d'interposition par le disque d'induction en position de repos et déplaçable en position d'effacement lorsque le disque d'induction est en position active sollicitée par les moyens de génération de champ magnétique variable.

Avantageusement, le déplacement du loquet est translatif, un organe de déplacement étant prévu pour venir en prise avec le loquet mobile pour le déplacer en translation de sa position d'interposition vers sa position d'effacement, cet organe de déplacement étant avantageusement intégré au dispositif de déverrouillage sous la forme d'un levier pivotant. En variante, le loquet peut être rotatif ou pivotant. Il peut aussi être relié au disque d'induction, par exemple par une biellette.

Selon une forme de réalisation pratique, le disque d'induction en position de repos peut comprendre une paroi de butée qui bloque le loquet mobile dans sa position d'interposition, le disque d'induction en position active comprenant un logement qui accueille le loquet déplacé en translation dans sa position d'effacement par l'organe de déplacement. De préférence, le loquet peut comprendre une tête d'interposition, un corps de guidage axial et un talon de butée, la tête d'interposition venant en contact de l'élément de fermeture amovible pour le bloquer, le talon de butée venant en contact avec la paroi de butée ou en position dans le logement, le corps de guidage axial formant avantageusement un profil de préhension pour l'organe de déplacement.

Selon un autre aspect de l'invention, le distributeur de produit fluide peut comprendre une platine de support formant un cadre de réception pour le disque d'induction et une cheminée de guidage axial pour le loquet, la cheminée de guidage axial débouchant dans le cadre, le cadre de réception étant avantageusement pourvu d'une tige définissant l'axe de rotation X pour le disque d'induction, d'une accroche pour des moyens élastiques sollicitant le disque d'induction en position de repos et d'un arrêt pour limiter la rotation du disque d'induction.

Selon un mode de réalisation préféré, le dispositif de déverrouillage peut comprendre plusieurs aimants permanents disposés parallèlement de manière alternée en polarité autour d'un axe de rotation Y, le dispositif de déverrouillage comprenant des moyens d'alignement d'axe aptes à favoriser l'alignement des deux axes X et Y, lorsque le distributeur de produit fluide est rapporté sur le dispositif de déverrouillage ou vice versa, le dispositif de déverrouillage comprenant ou étant associé à un moteur pour entrainer les aimants permanents autour de l'axe Y, le moteur tournant avantageusement à au moins 200 tours/minute et de préférence à environ 300 tours/minute.

L'esprit de l'invention réside dans le fait de déplacer un élément d'induction, qui est réalisé en un matériau conducteur amagnétique, au moyen d'un champ magnétique variable créé par un dispositif de déverrouillage, qui est distinct du distributeur.

L'invention sera maintenant plus amplement décrite en référence aux dessins joints qui donnent, à titre d'exemple non limitatif, un mode de réalisation de l'invention.

Sur les figures :
La figure 1 est une coupe transversale verticale à travers un distributeur de produit fluide selon l'invention,
La figure 2 est une vue latérale partiellement découpée du distributeur de produit fluide de la figure 1,
La figure 3 est une vue en perspective agrandie de la platine de support du distributeur des figures 1 et 2,
La figure 4 est une vue en perspective très fortement agrandie du loquet du distributeur des figure 1 et 2,
Les figures 5a et 5b sont des vues en perspective agrandie recto et verso du disque d'induction du distributeur des figures 1 et 2,
La figure 6a est une vue en perspective d'un dispositif de déverrouillage selon l'invention,
La figure 6b est une vue en perspective et en transparence partielle du dispositif de déverrouillage de la figure 6a,
La figure 6c est une représentation schématique visant à illustrer une configuration particulière pour les aimants du dispositif de déverrouillage des figures 6a et 6b,
La figure 7 est une vue en coupe transversale verticale à travers le dispositif de déverrouillage des figures 6a et 6b associé au distributeur des figures 1 et 2,
Les figures 8a et 8b sont des vues similaires aux figures 1 et 2 avec le dispositif de déverrouillage des figures 6a et 6b agissant sur le distributeur,
Les figures 9a et 9b sont des vues similaires à celles des figures 8a et 8b avec le dispositif de déverrouillage pleinement actionné, et
La figure 10 est une vue agrandie similaire aux figures 8b et 9b montrant le distributeur dans une configuration apte au retrait du capot du distributeur.

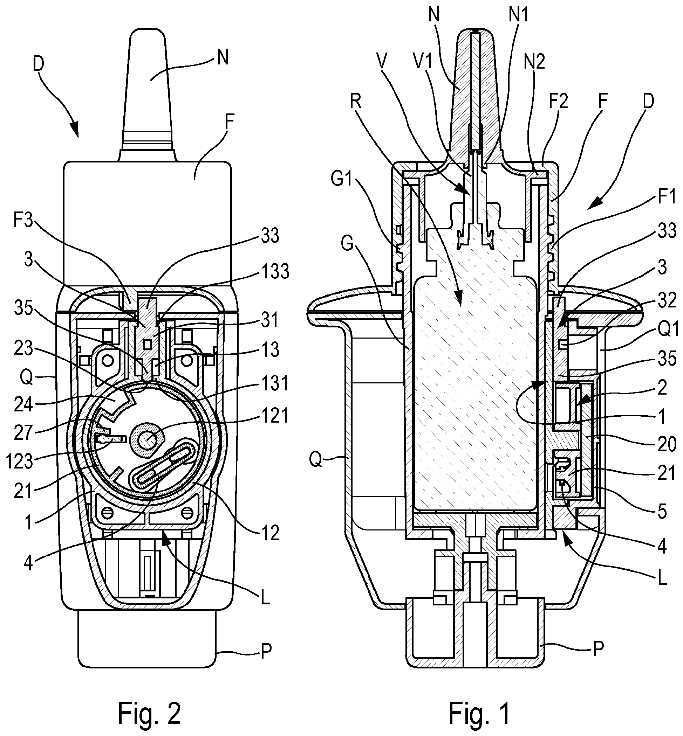

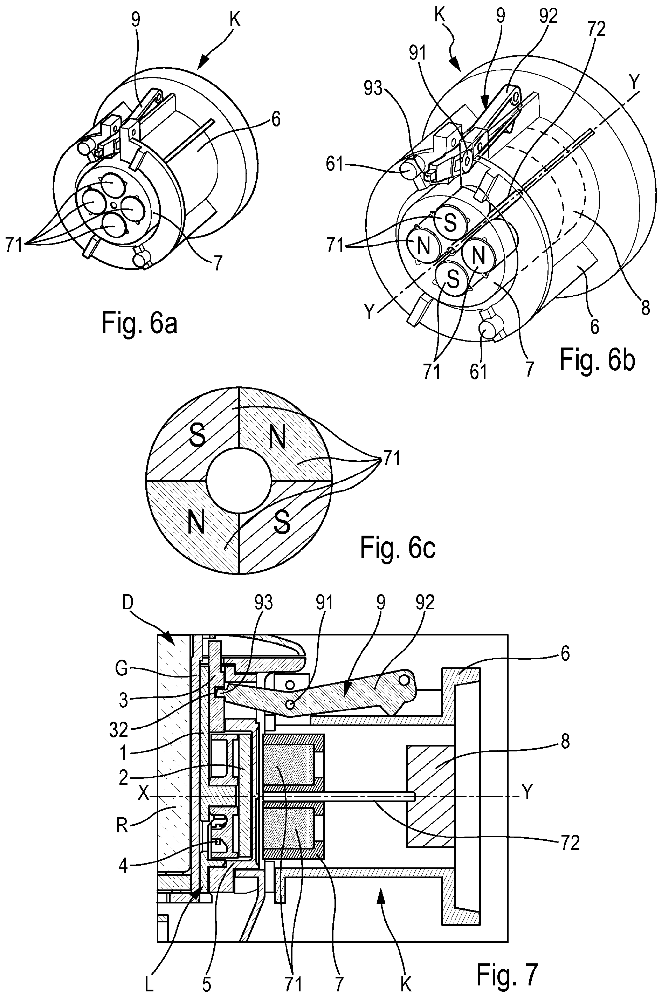

On se référera tout d'abord aux figures 1 et 2 pour décrire la structure générale d'un distributeur de produit fluide D selon l'invention. On se référera ensuite aux figures 6a, 6b, 6c et 7 pour décrire le dispositif de déverrouillage K de l'invention, qui est destiné à être associé au distributeur de produit fluide D pour le débloquer et autoriser ainsi une opération.

Le distributeur de produit fluide D des figures 1 et 2 est un distributeur nasal destiné à injecter du produit fluide, dosé ou non, dans une narine d'un patient. Il ne s'agit là que d'un exemple particulier, qui n'est en aucun cas limitatif. Tout autre type de distributeur de produit fluide pourrait être utilisé dans le cadre de la présente invention. Ainsi, le distributeur D comprend un réservoir de produit fluide R qui est ici équipé d'une valve de distribution V. A la place de la valve V, on aurait également pu utiliser une pompe ou tout autre dispositif de distribution. La valve V est coiffée d'un embout nasal N destiné à être inséré dans la narine du patient. L'embout nasal N comprend un manchon de raccordement N1 qui est engagé autour de l'extrémité libre de la tige de soupape V1 de la valve V. L'embout nasal N comprend également une collerette annulaire de maintien N2 qui permet de maintenir l'embout nasal N de manière fixe, comme on le verra ci-après. Le réservoir R est disposé à l'intérieur d'une gaine G qui forme un filetage externe G1 dans sa partie supérieure. La gaine G est ouverte à son extrémité inférieure pour le passage d'un poussoir P. Un capot de fermeture F est formé avec un filetage F1 et un rabat rentrant supérieur F2. Le filetage interne F1 est destiné à venir en prise filetée avec le filetage externe G1 de la gaine G. Lorsque le capot F est entièrement vissé sur la gaine G, son rabat rentrant supérieur F2 vient en prise avec la collerette annulaire de maintien N2 de l'embout nasal N et la maintient ainsi fixement en place.

On comprend déjà qu'un actionnement du poussoir P va déplacer le réservoir R à l'intérieur de la gaine G en laissant l'embout nasal N statique. Ce faisant, la valve V est contrainte dans son état ouvert, ce qui permet au produit fluide stocké dans le réservoir R d'être refoulé à travers la valve V et l'embout nasal N. Il s'agit là d'un fonctionnement tout à fait classique pour un distributeur nasal. On comprend aussi qu'il est possible d'accéder au réservoir R à l'intérieur de la gaine G en dévissant le capot de fermeture F. On peut ainsi procéder à son remplacement ou à l'insertion d'un réservoir différent.

Le distributeur D comprend également une coque Q qui enveloppe partiellement la gaine G, laisse un passage pour le poussoir P et sert également de surface d'accostage pour le capot de fermeture F. Cette coque externe Q comprend une fenêtre latérale Q1 dont la fonction sera donnée ci-après.

Ce distributeur D est pour l'instant d'une conception tout à fait classique, mais il incorpore en outre un système de blocage L, qui fait partie de l'invention.

On peut remarquer que le distributeur D comprend une platine de support 1, qui est montée sur une paroi latérale de la gaine G. Cette platine de support 1 sert de support à un disque d'induction 2 ainsi qu'à un loquet 3. Un couvercle 5 est monté sur la platine de support 1 en recouvrant entièrement le disque d'induction 2 et en laissant un accès au loquet 3. Le couvercle 5 est monté dans la fenêtre latérale Q1 de la coque Q.

Sur la figure 2, on peut remarquer que le loquet 3 comprend une tête d'interposition 33 contre laquelle bute un ergot de verrouillage F3 formé par le capot de fermeture vissable F. On comprend aisément que l'interposition de la tête 33 empêche le dévissage du capot F. Le distributeur D est donc dans une configuration de verrouillage, dans laquelle il est impossible d'accéder au réservoir R.

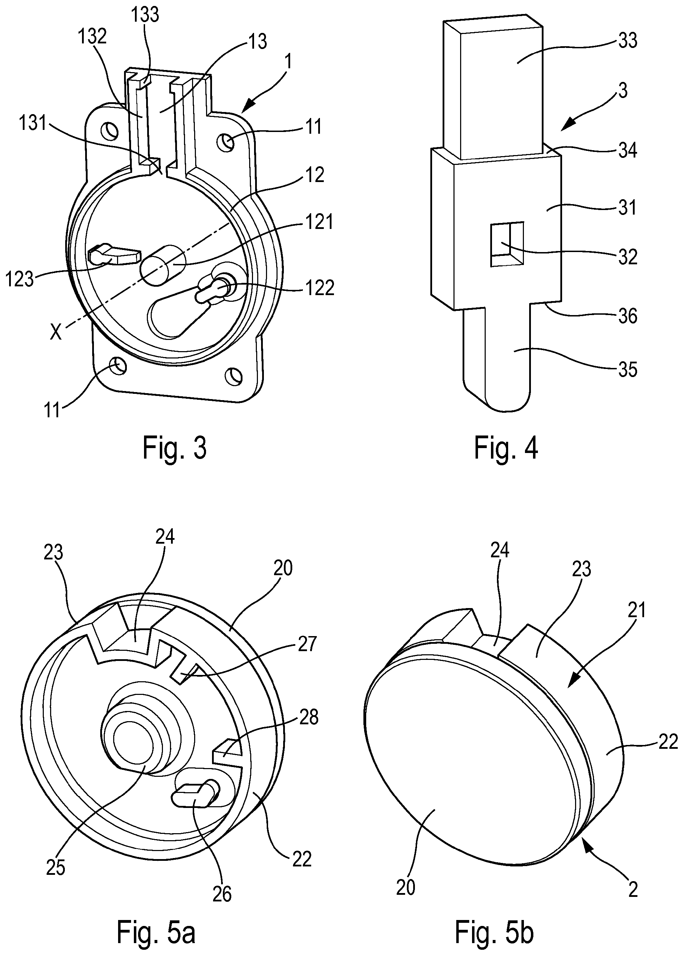

On se référera maintenant aux figures 3, 4, 5a et 5b pour décrire en détail la structure de la platine de support 1, du disque d'induction 2 et du loquet 3, qui constituent ensemble l'essentiel du système de blocage L de l'invention.

Sur la figure 3, on peut voir que la platine de support 1 comprend plusieurs trous de fixation 11, qui permettent son montage sur la gaine G, ou plus généralement sur une structure fixe du distributeur D. La platine de support 1 forme un cadre de réception 12 de forme annulaire, qui communique par un passage réduit inférieur 131 avec une cheminée verticale 13, définissant deux parois latérales opposées 133 et se terminant par une sortie réduite supérieure 133. Le cadre 12 forme intérieurement une tige 121 définissant un axe de rotation X, une accroche 122 pour un ressort 4, ainsi qu'un arrêt 123, qui s'étend sensiblement radialement. La platine de support 1 peut classiquement être réalisée par injection moulage de matière plastique.

Sur les figures 5a et 5b, on peut voir que le disque d'induction 2 comprend, dans cette forme de réalisation particulière, deux pièces distinctes qui sont solidaires l'une de l'autre, à savoir une pastille 20 réalisée en un matériau conducteur amagnétique et une roue 21 qui peut être réalisée par injection moulage de matière plastique. En variante, il est également possible de réaliser le disque d'induction 2 de manière monobloc, par exemple par moulage ou pressage d'un matériau conducteur amagnétique, tel que du cuivre ou de l'aluminium. Dans ce mode de réalisation particulier, la pastille 20 peut être de forme sensiblement cylindrique avec un chant plat et des faces planes opposées parallèles. La pastille 20 peut par exemple être collée ou encliquetée sur la roue 21. La roue 21 comprend un rebord sensiblement cylindrique 22, qui est interrompu au niveau d'un logement 24. Intérieurement, le rebord 22 forme deux profils de butée 27 et 28, qui s'étendent sensiblement radialement vers l'intérieur à partir du rebord 22. La roue 21 forme également un ancrage 26 pour un ressort de rappel 4, comme on le verra ci-après. Enfin, la roue 22 forme un moyeu 25 en son centre. On peut remarquer que le diamètre de la pastille 20 correspond sensiblement au diamètre du rebord 22 de la roue 21. Seul le logement 24 vient interrompre le rebord 22.

Sur la figure 4, on peut voir que le loquet 3 est une pièce monobloc, qui peut par exemple être réalisé par injection moulage de matière plastique. Le loquet 3 comprend un corps de guidage axial 31 qui est formé avec un profil de préhension 32 qui se présente sous la forme d'un évidement. A son extrémité supérieure, le corps de guidage se prolonge par la tête d'interposition 33 précédemment évoquée. On peut remarquer qu'un épaulement supérieur 34 réalise la jonction entre le corps 31 et la tête 33. De manière opposée, le corps 31 se prolonge vers le bas par un talon de butée 35. Là encore, un épaulement inférieur 36 fait la jonction entre le corps 31 et le talon 35.

Ces trois pièces du système de blocage L étant maintenant décrites, nous retournons aux figures 1 et 2 pour expliquer leur disposition et coopération au sein du distributeur D. On peut tout d'abord remarquer que la platine de support 1 est fixée sur la paroi latérale de la gaine G. Ensuite, on peut voir que le disque d'induction 2 est monté dans le cadre 12 de la platine 1. Le diamètre du disque d'induction 2 peut être légèrement inférieur à celui du cadre 12, de manière à réduire à la fois les jeux et les frottements. Le moyeu 25 de la roue 21 est engagé autour de la tige 121 de la platine 1. L'arrêt 123 est engagé entre les deux profils de butée 27 et 28, de manière à limiter la course angulaire du disque d'induction 2 dans le cadre 12. Un ressort 4, par exemple sous la forme d'une boucle élastique ou d'un ressort à boudin, est engagé autour de l'ancrage 26 et de l'accroche 122 de la platine 1. Ce ressort 4 sollicite le disque d'induction 2 dans le sens inverse des aiguilles d'une montre, de sorte que le profil de butée 27 vient en appui contre la raie 123. Ceci est visible sur la figure 2.

Quant au loquet, on peut voir que sa tête d'interposition 33 est située de manière adjacente à l'ergot de verrouillage F3 du capot à visser F. Ce corps 31 est engagé dans la cheminée 13 entre les deux parois latérales 132 et entre les deux passages réduits 131 et 133. On peut ainsi dire que le loquet 3 est prisonnier de la cheminée 13, tout en pouvant se déplacer en translation entre les deux passages réduits 131 et 133. La tête d'interposition 33 s'étend à travers le passage réduit supérieur 133 et le talon de butée 35 s'étend à travers le passage réduit inférieur 131. On peut remarquer sur les figures 1 et 2 que l'extrémité inférieure du talon de butée 35 est en contact avec le rebord 22 de la roue 21 en un point 23 qui est situé à proximité du logement 24. Dans cette position de blocage, l'épaulement supérieur 34 vient en butée contre le passage réduit supérieur 133, alors que l'épaulement inférieur 36 reste à distance du passage réduit inférieur 131. Le loquet 3 est donc parfaitement fixe dans cette position dite de verrouillage. Le dévissage du capot de fermeture F est empêché par la mise en butée de l'ergot F3 contre la tête d'interposition 33 du loquet 3. L'utilisateur ne peut pas accéder au réservoir R pour le remplacer.

En se référant aux figures 6a et 6b, on peut voir le dispositif de déverrouillage K de l'invention, qui permet d'entraîner le disque d'induction 2 en rotation sur un angle limité, permettant toutefois d'amener le logement 24 en regard du talon de butée 35 du loquet 3. Ce dispositif de déverrouillage K comprend un corps 6, qui peut être réalisé par injection moulage de matière plastique. Ce corps 6 présente une configuration générale sensiblement cylindrique avec un intérieur traversant. Le corps 6 contient un rotor 7 supportant plusieurs aimants permanents 71, avantageusement disposés avec des polarités alternées. Le rotor 7 est monté sur un arbre 72, qui peut être entraîné en rotation par un moteur 8. Dans une version minimaliste, le rotor 7 pourrait ne comprendre qu'un seul aimant disposé de manière excentrée par rapport à l'arbre 72 qui définit un axe de rotation Y. Dans cette version plus optimisée, les quatre aimants 71 sont disposés parallèlement à l'arbre 72, mais avec des polarités alternées, représentées par les majuscules N et S. Les aimants 71 peuvent se présenter sous la forme de petits plots cylindriques disposés côte à côte autour de l'arbre 72. En variante encore plus optimisée représentée sur la figure 6c, les quatre aimants 71 peuvent se présenter sous la forme de quartiers disposés de manière adjacents et formant ensemble un disque avec une ouverture centrale pour le passage de l'arbre 72. Sans sortir du cadre de l'invention, on peut bien entendu imaginer plus de quatre aimants 71, par exemple de six à douze. On comprend que le moteur 8 a pour fonction d'entraîner l'arbre 72 en rotation, entraînant ainsi le rotor 7 avec ses aimants 71 en rotation autour de l'axe de rotation Y. A la place du moteur 8, du rotor 7 avec de ses aimants 71 et de son arbre 72, on peut utiliser un jeu de solénoïdes, par exemple au nombre de quatre ou de six, disposés autour de l'axe Y et alimentés de manière alternée (+/-) de façon à réaliser un champ magnétique tournant. Ce mode de réalisation alternatif permet d'éviter l'utilisation d'un moteur et d'un rotor.

Le corps 6 est avantageusement pourvu de picots d'alignement 61 qui sont disposés autour du rotor. Leur fonction est de favoriser le positionnement du dispositif de déverrouillage K par rapport au distributeur D, ou vice versa.

Optionnellement, le dispositif de déverrouillage K comprend également un organe de déplacement 9, qui va permettre de déplacer le loquet 3 en translation dans sa cheminée 13, lorsque son talon de butée 35 sera disposé en face du logement 24 du disque d'induction 2, comme on le verra ci-après. Cet organe de déplacement 9 peut par exemple se présenter sous la forme d'un levier qui peut basculer autour d'un axe de pivot 91. Le levier comprend une partie d'actionnement 92, ainsi qu'une partie d'engagement formant un nez d'insertion 93, adapté à venir s'insérer dans l'évidement 32 du loquet 3. Ce moyen de déplacement 9 pourrait également être distinct et séparé du dispositif de déverrouillage K.

Sur la figure 7, le dispositif de déverrouillage K est associé au distributeur D, dans une disposition telle que les axes de rotation X et Y sont alignés et maintenant confondus. L'alignement de ces axes est favorisé par l'engagement des picots d'alignement 61 du corps 6 des logements correspondants du distributeur D, par exemple au niveau du couvercle 5. Le rotor 7 est presque en contact du couvercle 5, de sorte que les aimants 71 sont disposés à proximité du disque d'induction 2, de manière à optimiser l'action des aimants 71 sur le disque 2. Le nez 93 du levier 9 est engagé à l'intérieur de l'évidement 32 du loquet 3. Le moteur 8 n'est pas encore activé, de sorte que le disque d'induction 2 est dans la position représentée sur la figure 2, avec le talon de butée 35 du loquet 3 en contact du rebord 22 de la roue 21 en 23.

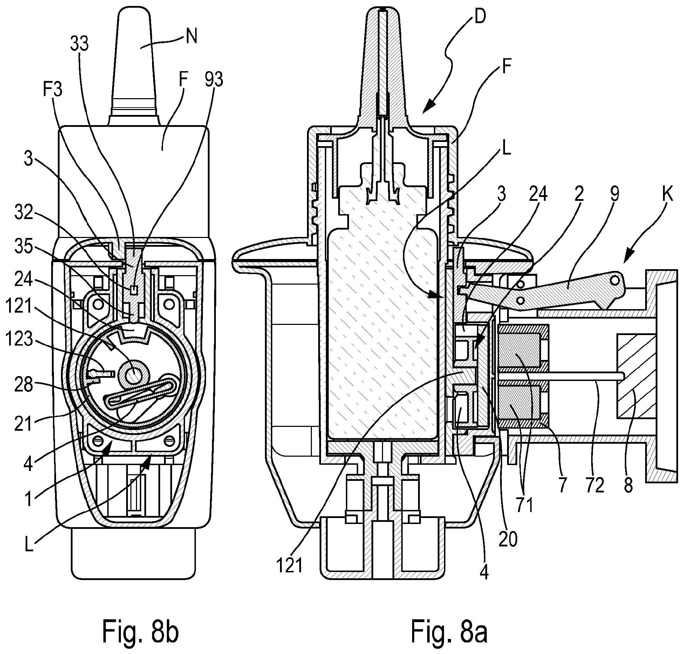

Les figures 8a et 8b illustrent la configuration du distributeur D lorsque les aimants 71 sont entraînés par le rotor 7 en rotation dans le sens horaire. Comme expliqué ci-dessus, la rotation du rotor 7 génère un champ magnétique variable qui va générer une force électromotrice sur le disque d'induction 2, ou plus particulièrement sur sa pastille conductrice amagnétique 20. Le disque d'induction 2 va ainsi être entraîné en rotation dans le sens horaire, à l'encontre de la force exercée par le ressort 4 pour parvenir dans la configuration représentée sur la figure 8b. On peut voir que le logement 24 est maintenant situé juste en dessous du talon de butée 35 et que le profil de butée 28 est en contact de l'arrêt 123. Le capot F est toutefois encore bloqué en rotation du fait de l'interposition de la tête 33 du loquet 3.

Le couple engendré sur le disque d'induction 2 par la rotation du rotor 7 est dépendant de nombreux paramètres comme par exemple :
- le champ magnétique créé par chaque aimant 71, qui dépend de sa qualité,
- le nombre d'aimants 71,
- la disposition des aimants 71 sur le rotor 7,
- la géométrie des aimants,
- l'épaisseur de la pastille 20,
- la résistivité électrique du matériau du disque d'induction, le meilleur étant le cuivre, ou encore
- la distance entre la surface du disque d'induction 2 et les aimants 71.

Afin de créer un couple suffisant sur le disque d'induction 2, il est nécessaire que le rotor 7 tourne à une vitesse élevée : au moins 200 tours/minute et de préférence 300 tour/min, même en optimisant tous les paramètres listés ci-dessus. Cette vitesse de rotation élevée nécessaire participe et renforce aussi l'inviolabilité du système de blocage.

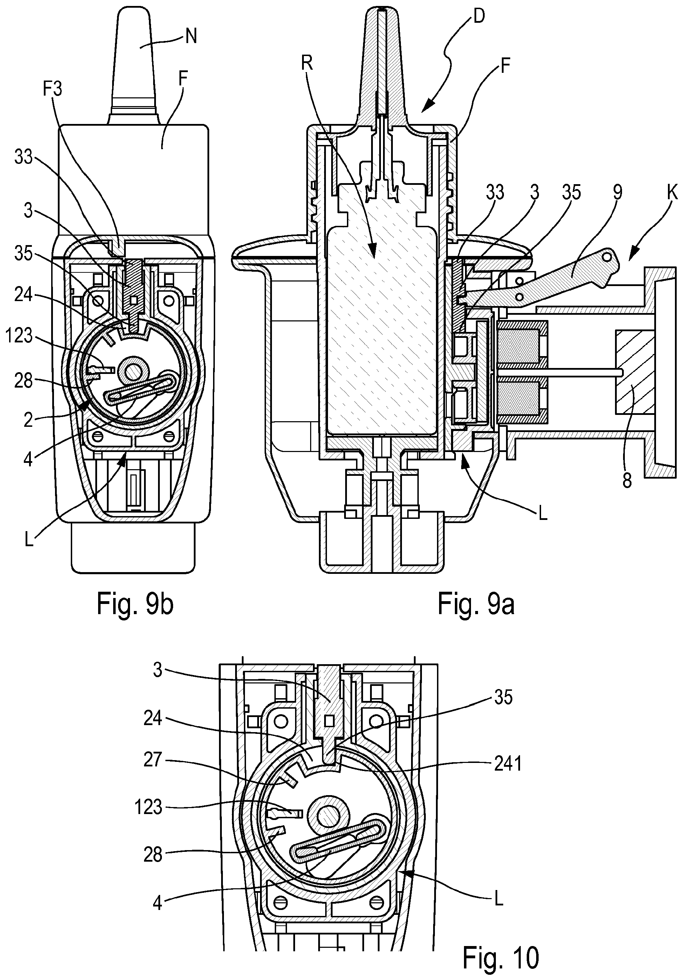

Sur les figures 9a et 9b, on peut voir que le levier 9 a été actionné, de manière à déplacer le loquet 3 entre translation vers le bas à l'intérieur de sa cheminée 13. Le talon de butée 35 est maintenant engagé à l'intérieur du logement 24 et la tête d'interposition 33 est maintenant disposée en dessous de l'ergot de verrouillage F3. Le moteur 8 est toujours alimenté de manière à conserver l'orientation angulaire du disque d'induction 2. Toutefois, dès l'engagement du talon de butée 35 à l'intérieur du logement 24, l'alimentation du moteur 8 peut être coupée, ce qui sollicite le disque d'induction 2 dans le sens inverse des aiguilles d'une montre sur une très faible course, puisque le talon de butée 35 va venir en butée contre une paroi latérale 241 du logement 24, comme représenté sur la figure 10, sous l'action du ressort 4.

Dès lors, le capot F peut être dévissé et le réservoir R peut être remplacé par un autre du même type ou d'un type différent. Le capot F peut ensuite être remis en place par vissage sur la gaine G. Pour finir, le loquet 3 peut être déplacé dans sa configuration de verrouillage de la figure 2, à l'aide du levier 9 ou à l'aide d'un autre ustensile.

Bien que l'invention ait été décrite en référence à un capot de fermeture à visser, on peut très bien imaginer le système de blocage L de l'invention agir sur n'importe quel organe mobile d'un distributeur, que cet organe se déplace en rotation ou en translation. Le système de blocage L de l'invention, qui est constitué ici par la platine 1, le disque d'induction 2 le loquet 3 et le ressort 4, peut dans d'autres modes de réalisation être réduit à une platine et à un disque d'induction rotatif qui agit directement sur l'organe mobile, ou encore à une platine et un loquet déplaçable (de par le disque d'induction) en translation et qui agit directement sur l'organe mobile.

Sans sortir du cadre de l'invention, la platine de support pourrait être intégrée à une pièce du distributeur, comme par exemple la gaine G. L'organe de déplacement 9 pourrait être actionné par un moteur. A la place de deux profils de butée 27 et 28 sur le disque d'induction 2 et un arrêt 123 sur la platine 1, on peut prévoir un profil de butée sur le disque et deux arrêts sur la platine.

Grâce à l'invention, on dispose d'un distributeur dont le système de blocage est inaccessible et totalement incompréhensible et dont le dispositif de déverrouillage met en œuvre des moyens électromagnétiques spécifiques peu courants.

## Revendications

1. Ensemble comprenant :
- un distributeur de produit fluide (D) comprenant un organe mobile (F) et un système de blocage (L) commutable entre une position de blocage dans laquelle l'organe mobile (F) est bloqué et une position de libération dans laquelle l'organe mobile (F) est libre,
- un dispositif de déverrouillage (K), distinct du distributeur de produit fluide (D), apte à commuter le système de blocage (L) entre la position de blocage et la position de libération, ce dispositif de déverrouillage (K) agissant sans contact, à distance, par génération de courant de Foucault, dans lequel le système de blocage (L) comprend un élément d'induction mobile (2) et le dispositif de déverrouillage (K) comprend des moyens de génération de champ magnétique variable (7) induisant une force électromotrice sur l'élément d'induction mobile (2) qui permet de commuter le système de blocage (L) de sa position de blocage vers sa position de libération,
**caractérisé en ce que** l'élément d'induction mobile est un disque d'induction (2) rotatif autour d'une axe X et les moyens de génération de champ magnétique variable (7) comprennent au moins un aimant permanent (71) entrainé en rotation autour d'un axe Y ou un jeu de solénoïdes disposés autour d'un axe Y et alimentés de manière alternée de façon à réaliser un champ magnétique tournant, les axes X et Y étant alignés lorsque le dispositif de déverrouillage (K) est rapporté sur le distributeur de produit fluide (D) ou vice versa, de sorte les moyens de génération de champ magnétique variable (7) induisent ainsi un mouvement de rotation au disque d'induction (2) à partir d'une position de repos vers une position active.

2. Ensemble selon la revendication 1, dans lequel le disque d'induction (2) est sollicité en position de repos par des moyens élastiques (4).

3. Ensemble selon la revendication 1 ou 2, dans lequel le disque d'induction (2) comprend au moins un profil de butée (27, 28) pour limiter sa rotation entre la position de repos et la position active.

4. Ensemble selon l'une quelconque des revendications précédentes, dans lequel le système de blocage (L) comprend en outre un loquet (3) déplaçable entre une position d'interposition dans laquelle le loquet (3) bloque l'organe mobile (F) et une position d'effacement dans laquelle l'organe mobile (F) est libre, le loquet (3) étant bloqué dans la position d'interposition par le disque d'induction (2) en position de repos et déplaçable en position d'effacement lorsque le disque d'induction (2) est en position active sollicitée par les moyens de génération de champ magnétique variable (7).

5. Ensemble selon la revendication 4, dans lequel le déplacement du loquet (3) est translatif, un organe de déplacement (9) étant prévu pour venir en prise avec le loquet (3) pour le déplacer en translation de sa position d'interposition vers sa position d'effacement, cet organe de déplacement (9) étant avantageusement intégré au dispositif de déverrouillage (K) sous la forme d'un levier pivotant.

6. Ensemble selon la revendication 4 ou 5, dans lequel le disque d'induction (2) en position de repos comprend une paroi de butée (23) qui bloque le loquet (3) dans sa position d'interposition, le disque d'induction (2) en position active comprenant un logement (24) qui accueille le loquet (3) déplacé en translation dans sa position d'effacement par l'organe de déplacement (9).

7. Ensemble selon la revendication 6, dans lequel le loquet (3) comprend une tête d'interposition (33), un corps de guidage axial (31) et un talon de butée (35), la tête d'interposition (33) venant en contact de l'organe mobile (F) pour le bloquer, le talon de butée (35) venant en contact avec la paroi de butée (23) ou en position dans le logement (24), le corps de guidage axial (31) formant avantageusement un profil de préhension (32) pour l'organe de déplacement (9).

8. Ensemble selon l'une quelconque des revendications 4 à 7, dans lequel le distributeur de produit fluide (D) comprend une platine de support (1) formant un cadre de réception (12) pour le disque d'induction (2) et une cheminée de guidage axial (13) pour le loquet (3), la cheminée de guidage axial (13) débouchant dans le cadre de réception (12), le cadre de réception (12) étant avantageusement pourvu d'une tige (121) définissant l'axe de rotation X pour le disque d'induction (2), d'une accroche (122) pour des moyens élastiques (4) sollicitant le disque d'induction (2) en position de repos et d'un arrêt (123) pour limiter la rotation du disque d'induction (2).

9. Ensemble selon l'une quelconque des revendications précédentes, dans lequel le dispositif de déverrouillage (K) comprend plusieurs aimants permanents (71) disposés parallèlement de manière alternée en polarité autour d'un axe de rotation Y, le dispositif de déverrouillage (K) comprenant des moyens d'alignement d'axe (61) aptes à favoriser l'alignement des deux axes X et Y, lorsque le distributeur de produit fluide (D) est rapporté sur le dispositif de déverrouillage (K) ou vice versa, le dispositif de déverrouillage (K) comprenant un moteur (8) pour entrainer les aimants permanents (71) autour de l'axe Y, le moteur (8) tournant avantageusement à au moins 200 tours/minute et de préférence à environ 300 tours/minute.

## Patentansprüche

1. Anordnung, umfassend:
- einen Spender für fluide Produkte (D), umfassend ein bewegliches Element (F) und ein Arretiersystem (L), das zwischen einer Arretierposition, in der das bewegliche Element (F) arretiert ist, und einer Freigabeposition, in der das bewegliche Element (F) frei ist, umschaltbar ist,
- eine Entriegelungsvorrichtung (K), die sich vom Spender für fluide Produkte (D) unterscheidet, die imstande ist, das Arretiersystem (L) zwischen der Arretierposition und der Freigabeposition umzuschalten, wobei diese Entriegelungsvorrichtung (K) berührungslos aus der Ferne durch Erzeugung eines Foucault-Stroms wirkt, wobei das Arretiersystem (L) ein bewegliches Induktionselement (2) umfasst und die Entriegelungsvorrichtung (K) Mittel zur Erzeugung eines variablen Magnetfelds (7) umfasst, das eine elektromotorische Kraft auf das bewegliche Induktionselement (2) induziert, die gestattet, das Arretiersystem (L) aus seiner Arretierposition in seine Freigabeposition umzuschalten,
**dadurch gekennzeichnet, dass** das bewegliche Induktionselement eine Induktionsscheibe (2) ist, die um eine Achse X dreht und die Mittel zur Erzeugung eines variablen Magnetfelds (7) mindestens einen Permanentmagneten (71) umfassen, der um eine Achse Y rotatorisch angetrieben wird oder einen Satz von Solenoiden, die um eine Achse Y angeordnet sind und alternierend versorgt werden, um ein rotierendes Magnetfeld zu erzeugen, wobei die Achsen X und Y ausgerichtet sind, wenn die Entriegelungsvorrichtung (K) am Spender für fluide Produkte (D) angebracht ist oder umgekehrt, so dass die Mittel zur Erzeugung eines variablen Magnetfelds (7) somit eine Drehbewegung der Induktionsscheibe (2) aus einer Ruheposition in eine aktive Position induzieren.

2. Anordnung nach Anspruch 1, wobei die Induktionsscheibe (2) durch elastische Mittel (4) in die Ruheposition gezwungen wird.

3. Anordnung nach Anspruch 1 oder 2, wobei die Induktionsscheibe (2) mindestens ein Anschlagprofil (27, 28) aufweist, um ihre Drehung zwischen der Ruheposition und der aktiven Position zu begrenzen.

4. Anordnung nach einem der vorstehenden Ansprüche, wobei das Arretiersystem (L) ferner einen Riegel (3) umfasst, der zwischen einer Zwischenposition, in der der Riegel (3) das bewegliche Element (F) arretiert, und einer zurückgezogenen Position, in der das bewegliche Element (F) frei ist, bewegbar ist, wobei der Riegel (3) in der Zwischenposition durch die Induktionsscheibe (2) in Ruheposition arretiert ist und in die zurückgezogene Position bewegbar ist, wenn die Induktionsscheibe (2) in der aktiven Position ist, die durch die Mittel zur Erzeugung eines variablen Magnetfelds (7) erzwungen wird.

5. Anordnung nach Anspruch 4, wobei die Bewegung des Riegels (3) translatorisch ist, wobei ein Verstellorgan (9) vorgesehen ist, um mit dem Riegel (3) in Eingriff zu kommen, um ihn translatorisch aus seiner Zwischenposition in seine zurückgezogene Position zu bewegen, wobei dieses Verstellorgan (9) in vorteilhafter Weise in die Entriegelungsvorrichtung (K) in Form eines schwenkbaren Hebels integriert ist.

6. Anordnung nach Anspruch 4 oder 5, wobei die Induktionsscheibe (2) in Ruheposition eine Anschlagwand (23) umfasst, die den Riegel (3) in seiner Zwischenposition arretiert, wobei die Induktionsscheibe (2) in ihrer aktiven Position eine Aufnahme (24) umfasst, die den durch das Verstellorgan (9) in seine zurückgezogene Position translatorisch bewegten Riegel (3) aufnimmt.

7. Anordnung nach Anspruch 6, wobei der Riegel (3) einen Zwischenkopf (33), einen axialen Führungskörper (31) und einen Anschlagabsatz (35) umfasst, wobei der Zwischenkopf (33) mit dem beweglichen Element (F) in Kontakt kommt, um es zu arretieren, wobei der Anschlagabsatz (35) mit der Anschlagwand (23) in Kontakt kommt oder in Position in der Aufnahme (24), wobei der axiale Führungskörper (31) in vorteilhafter Weise ein Greifprofil (32) für das Verstellorgan (9) bildet.

8. Anordnung nach einem der Ansprüche 4 bis 7, wobei der Spender für fluide Produkte (D) eine Trägerplatte (1) umfasst, die einen Empfangsrahmen (12) für die Induktionsscheibe (2) und eine axiale Führungsbahn (13) für den Riegel (3) bildet, wobei die axiale Führungsbahn (13) in den Empfangsrahmen (12) mündet, wobei der Empfangsrahmen (12) in vorteilhafter Weise mit einer Stange (121), die die Drehachse X für die Induktionsscheibe (2) definiert, einer Halterung (122) für elastische Mittel (4), die die Induktionsscheibe (2) in Ruheposition zwingen, und einer Arretierung (123) zur Begrenzung der Drehung der Induktionsscheibe (2) versehen ist.

9. Anordnung nach einem der vorstehenden Ansprüche, wobei die Entriegelungsvorrichtung (K) mehrere Permanentmagnete (71) umfasst, die parallel und mit alternierender Polarität um eine Drehachse Y angeordnet sind, wobei die Entriegelungsvorrichtung (K) Achsenausrichtungsmittel (61) umfasst, die imstande sind, die Ausrichtung der beiden Achsen X und Y zu unterstützen, wenn der Spender für fluide Produkte (D) an der Entriegelungsvorrichtung (K) angebracht ist oder umgekehrt, wobei die Entriegelungsvorrichtung (K) einen Motor (8) zum Antreiben der Permanentmagnete (71) um die Achse Y umfasst, wobei der Motor (8) in vorteilhafter Weise mit mindestens 200 Umdrehungen/Minute und vorzugsweise mit etwa 300 Umdrehungen/Minute dreht.

## Claims

1. Assembly comprising:
- a fluid product dispenser (D) comprising a movable member (F) and a locking system (L) that is switchable between a locking position, in which the movable member (F) is locked and a release position, in which the movable member (F) is unlocked,
- an unlocking device (K), separate from the fluid product dispenser (D), that is able to switch the locking system (L) between the locking position and the release position, this unlocking device (K) being an eddy-current-based contactless and remote unlocking device, wherein the locking system (L) comprises a movable induction element (2) and the unlocking device (K) comprises variable magnetic field generation means (7) inducing an electromotive force on the movable induction element (2) which makes it possible to switch the locking system (L) from its locking position to its release position,
**characterised in that** the movable induction element is an induction disc (2) rotating about an axis X and the variable magnetic field generation means (7) comprise at least one permanent magnet (71) rotated about an axis Y or a set of solenoids disposed about an axis Y and alternately powered so as to produce a rotating magnetic field, the axes X and Y being aligned when the unlocking device (K) is added to the fluid product dispenser (D) or vice versa, such that the variable magnetic field generation means (7) thus induce a rotary movement to the induction disc (2) from a rest position to an active position.

2. Assembly according to claim 1, wherein the induction disc (2) is urged into the rest position by resilient means (4).

3. Assembly according to claim 1 or 2, wherein the induction disc (2) comprises at least one abutment profile (27, 28) to limit its rotation between the rest position and the active position.

4. Assembly according to any one of the preceding claims, wherein the locking system (L) further comprises a catch (3) that is movable between an interposition position in which the catch (3) locks the movable member (F) and a release position in which the movable member (F) is unlocked, the catch (3) being locked in the interposition position by the induction disc (2) in the rest position and movable in the release position when the induction disc (2) is in the active position urged by the variable magnetic field generation means (7).

5. Assembly according to claim 4, wherein the movement of the catch (3) is translative, a movement member (9) being provided to engage with the catch (3) to move it translatably from its interposition position to its release position, this movement member (9) being advantageously integrated in the unlocking device (K) in the form of a pivoting lever.

6. Assembly according to claim 4 or 5, wherein the induction disc (2) in the rest position comprises an abutment wall (23) which locks the catch (3) in its interposition position, the induction disc (2) in the active position comprising a housing (24) which receives the catch (3) translatably moved into its release position by the movement member (9).

7. Assembly according to claim 6, wherein the catch (3) comprises an interposition head (33), an axial guiding body (31) and an abutment stub (35), the interposition head (33) coming into contact with the movable member (F) to lock it, the abutment stub (35) coming into contact with the abutment wall (23) or in position in the housing (24), the axial guiding body (31) advantageously forming a gripping profile (32) for the movement member (9).

8. Assembly according to any one of claims 4 to 7, wherein the fluid product dispenser (D) comprises a support plate (1) forming a receiving frame (12) for the induction disc (2) and an axial guiding funnel (13) for the catch (3), the axial guiding funnel (13) opening into the receiving frame (12), the receiving frame (12) being advantageously provided with a rod (121) defining the axis of rotation X for the induction disc (2), with a hook (122) for the resilient means (4) urging the induction disc (2) into the rest position and a stop (123) to limit the rotation of the induction disc (2).

9. Assembly according to any one of the preceding claims, wherein the unlocking device (K) comprises several permanent magnets (71) disposed alternately parallel in polarity about an axis of rotation Y, the unlocking device (K) comprising axis alignment means (61) that are able to favour the alignment of the two axes X and Y, when the fluid product dispenser (D) is added to the unlocking device (K) or vice versa, the unlocking device (K) comprising a motor (8) to drive the permanent magnets (71) about the axis Y, the motor (8) rotating advantageously at at least 200 rotations per minute, and preferably at around 300 rotations per minute.
